# EUROPEAN PATENT APPLICATION

(11) **EP 4 124 377 A1**
(43) Date of publication of application: **01.02.2023**
(21) Application number: 21188313.7
(22) Date of filing: 28.07.2021
(51) Int. Cl.: B01D 61/00, B01D 61/02, B01D 61/58, A61M 1/16, C02F 1/44

(54) **WASTEWATER TREATMENT FOR DIALYSIS**

(71) Applicant: Greentec Dialysis GmbH, 69115 Heidelberg (DE)
(72) Inventor: The designation of the inventor has not yet been filed
(74) Representative: Maiwald GmbH

(57) **Abstract**

A wastewater treatment system (100) for treating and/or purifying wastewater from one or more dialysis devices (300) is provided. The system comprises at least one inlet (102) configured to receive wastewater from one or more dialysis devices, a container (120) configured to store a draw solution containing a solvent for forward osmosis, a forward osmosis device (110) fluidly coupled to the at least one inlet (102) to receive wastewater from the at least one inlet and fluidly coupled to the container (120) to receive the draw solution from the container, and a reverse osmosis device (130) fluidly coupled to the container (120) and/or the forward osmosis device (110), the reverse osmosis device being configured to generate purified water based on reverse osmosis. Therein, the forward osmosis device (110) is configured to extract, based on forward osmosis using the draw solution, water molecules from the received wastewater into the draw solution, thereby decreasing a concentration of the solvent in the draw solution. Further, the reverse osmosis device (130) is configured to extract, based on reverse osmosis, the purified water from the draw solution with decreased solvent concentration, thereby increasing the concentration of the solvent in the draw solution.

## Description

### Technical Field

The present disclosure generally relates to the field of dialysis. In particular, the present disclosure relates to a wastewater treatment system for treating and/or purifying wastewater from one or more dialysis devices. Further, the present disclosure relates to a method of treating and/or purifying wastewater from one or more dialysis devices with such system.

### Technical Background

Generally, dialysis is used to purify blood from patients whose kidneys may be functionally disturbed and/or may have failed. In dialysis, the blood to be purified is extracted from a patient and supplied to a dialysis device, where the blood usually passes a semipermeable membrane on one side of the membrane with a sterile solution flowing on another side of the membrane. The semipermeable membrane is also referred to as dialyzer. Due to a gradient in concentration of substances and/or constituents in the fluids on the two sides of the membrane, an osmotic pressure is generated that can cause certain substances and/or constituents to pass from the blood through the semipermeable membrane into the solution. For this reason, the solution is used to pull substances through the membrane is also referred to as draw solution. Typically, substances and/or constituents extracted from the patient's blood by dialysis can only hardly be removed from a patient's blood by the patient itself due to an organ failure or malfunction. However, too high concentrations of certain substances and/or constituents can be troublesome for the patient and adversely affect its health status.

Dialysis is a common treatment procedure today that can help a large number of people. Because a single dialysis treatment of a patient lasts for several hours but has to be repeated regularly, dialysis is usually performed in dedicated dialysis centers where a large number of dialysis stations with appropriate dialysis machines, devices, apparatuses and/or systems is provided.

Dialysis devices typically prepare, inter alia, the sterile solution into which the interfering substances and/or constituents are to be extracted from the blood. These dialysis solutions are typically prepared from water, in particular drinking water and/or fresh water, which was previously purified by reverse osmosis. Such purified water (also referred to as dialysis water, pure water and/or permeate) is usually heated to a body temperature of around 37°C in the dialysis device, mixed with an electrolyte, which can optionally be mixed from different solutions in the dialysis device, to create a dialysis solution with favorable, predetermined and/or defined conditions for the patient.

The amounts and quantities of water treated or prepared in this manner to supply the purified dialysis water to dialysis devices are considerably high. For this reason, dialysis centers or institutions usually use a central water treatment system providing the purified water via respective feed lines to the dialysis devices. As mentioned above, the dialysis devices then usually prepare the dialysis solution by adding electrolyte to the purified water, heating the solution and passing it along the semipermeable to extract substances and/or constituents from the patient's blood passed along an opposite side of the membrane. The used dialysis solution or permeate is then usually fed into the public wastewater system. Consequently, significant amounts of fresh water can be required for dialysis and the overall water consumption in dialysis can be considerably high.

### Summary

For the reasons set out hereinabove, it may be desirable to provide for an improved system and method of treating and/or purifying wastewater from one or more dialysis devices.

This is achieved by the subject matter of the independent claims, wherein further embodiments are defined in the dependent claims and the following description.

According to an aspect of the present disclosure, there is provided wastewater treatment system (also referred to as system herein) for treating and/or purifying wastewater from one or more dialysis devices. The system comprises at least one inlet configured to receive wastewater from one or more dialysis devices, and a container configured to store and/or storing a draw solution containing a solvent for forward osmosis. The system further includes a forward osmosis device fluidly coupled to (and/or in fluid communication with) the at least one inlet to receive wastewater from the at least one inlet and fluidly coupled to (and/or in fluid communication with) the container to receive the draw solution from the container. Accordingly, the forward osmosis device can be coupled and/or interconnected with the container and the inlet. The system further includes a reverse osmosis device fluidly coupled to (and/or in fluid communication with) the container and/or the forward osmosis device, wherein the reverse osmosis device is configured to generate purified water based on reverse osmosis. Therein, the forward osmosis device is configured to extract, based on forward osmosis using the draw solution, water molecules and/or water from the received wastewater into the draw solution, thereby decreasing a concentration of the solvent in the draw solution. Accordingly, by means of the forward osmosis device, the draw solution in the container and/or received from the container can be diluted based on forward osmosis extracting water from the wastewater, while other constituents and/or substances can remain in the wastewater. Further, the reverse osmosis device is configured to extract, based on reverse osmosis, the purified water from the draw solution with decreased solvent concentration, thereby increasing the concentration of the solvent in the draw solution. The purified water generated by the reverse osmosis device can then be used for dialysis and/or can be supplied to one or more dialysis devices for dialysis treatment.

Accordingly, the proposed system can be considered as a two stage system with a forward osmosis stage and a reverse osmosis stage, which can be performed simultaneously or sequentially. Using such two-stage process combining forward osmosis and reverse osmosis, wastewater generated by the one or more dialysis devices can be purified and a significant amount of water contained in the wastewater can be extracted therefrom, purified and re-used for dialysis. As mentioned hereinabove, conventionally, wastewater of dialysis devices is usually conducted, purged and/or fed into the public wastewater system and cannot be re-used. This waste of water can be significantly mitigated, reduced or even avoided by the system according to the present invention

According to an embodiment, the forward osmosis device includes at least one semipermeable membrane, wherein the forward osmosis device is configured to pass and/or guide the wastewater along a first side of the membrane and to pass and/or guide the draw solution along a second side of the membrane opposite to the first side, thereby extracting water molecules from the wastewater into the draw solution through the membrane. Therein, the concentration of solvent in the draw solution may be sufficiently high to generate an osmotic pressure resulting in a flow of water from the wastewater through the semipermeable membrane and into the draw solution. The remaining wastewater may optionally be fed back into a wastewater container, for example, and/or it may be disposed.

According to an embodiment, the reverse osmosis device includes at least one semipermeable membrane, wherein the reverse osmosis device is configured to receive the draw solution from the container, increase a pressure of the draw solution and pass the pressurized draw solution along a first side of the membrane, such that osmosis is reversed, and water molecules are extracted from the pressurized draw solution through the membrane. Accordingly, purified and/or pure water can be generated by means of the reverse osmosis device and the draw solution stored in the container and/or generated by the forward osmosis device. By increasing the pressure of the draw solution passed and/or guided along the one side of the membrane, water can be extracted therefrom against the osmotic pressure. In other words, by increasing the pressure of the draw solution, water and/or water molecules can be forced to traverse the membrane against the osmotic pressure and/or a concentration gradient that acts in opposite direction.

Summarizing the above, wastewater supplied via the inlet from one or more dialysis devices can be fed to the forward osmosis device, wherein water molecules (also referred to as water herein), can be extracted from the wastewater through the semipermeable membrane of the forward osmosis device and into the draw solution. This in turn leads to a decrease in the concentration of solvent in the draw solution. The draw solution with decreased solvent concentration (or decreased concentration of solvent) can then be supplied to the container, thereby diluting the draw solution in the container. In order to keep the forward osmosis in the forward osmosis device running, this decrease in solvent concentration should preferably be compensated for. This compensation of the dilution of the draw solution is, according to the present disclosure, provided and/or accomplished by the reverse osmosis device, which receives the draw solution with decreased solvent concentration generated by the forward osmosis device and extracts purified water therefrom by reverse osmosis. It should be noted that the draw solution with decreased solvent concentration generated by the forward osmosis device can be directly supplied from the forward osmosis device to the reverse osmosis. Alternatively or additionally, at least a part of or the entire draw solution with decreased solvent concentration generated by the forward osmosis device can be supplied to the container and the reverse osmosis device can be configured to use the draw solution from the container to perform reverse osmosis.

As used herein, the draw solution may refer to or denote a fluid and/or liquid comprising the solvent. Further, the "solvent" may refer to, denote, and/or comprise an ionic substance or compound. It should be noted that the "solvent concentration" as used herein can refer to an "ion concentration". Hence, solvent concentration can be used synonymously with ion concentration herein.

Generally, the concentration of solvent or ion concentration in the draw solution may be sufficiently high to extract water from the wastewater through the membrane of the forward osmosis device. Accordingly, the solvent concentration and/or ion concentration in the draw solution should be higher than in the wastewater in order to generate the osmotic pressure resulting in the flow of water from the wastewater through the semipermeable membrane and into the draw solution. Generally, any suitable or appropriate draw solution and/or solvent can be used for this purpose.

As used herein, the at least one inlet of the system may be fluidly coupled to and/or may be in fluid communication with the forward osmosis device. The at least one inlet may, for example, be arranged at a wastewater container or tank for storing wastewater received from the one or more dialysis devices. However, the inlet may also be directly coupled to one or more dialysis devices. When referring to a single inlet hereinabove and hereinbelow, also a plurality of inlets is included. The same applies to the "at least one outlet" of the system referred to herein.

Likewise, any reference herein to a single container, such as the container for storing the draw solution and/or a wastewater container for storing the wastewater from the dialysis devices, includes a plurality of containers.

As used herein, the draw solution with decreased solvent concentration may refer to or denote the draw solution after being generated, processed and/or used by the forward osmosis device. Similarly, the draw solution with increased solvent concentration can refer to or denote the draw solution after being generated, processed and/or used by the reverse osmosis device.

According to an embodiment, the system is configured to supply the draw solution with increased solvent concentration generated by the reverse osmosis device to the container and/or to the forward osmosis device. In other words, the draw solution with increased solvent concentration generated by the reverse osmosis device can be fed back into the container, such that it can be re-used by the forward osmosis device for forward osmosis. Alternatively or additionally, the draw solution with increased solvent concentration generated by the reverse osmosis device can be directly supplied to the forward osmosis device, such that it can be re-used by the forward osmosis device. Generally, this allows to re-use the draw solution in a closed loop between the forward osmosis device and the reverse osmosis device, and optionally the container. Accordingly, the container for storing the draw solution may, at least in certain embodiments, be fluidly coupled between the forward osmosis device and the reverse osmosis device, e.g. such that draw solution with decreased solvent concentration can be supplied from the forward osmosis device via the container to the reverse osmosis device. Alternatively or additionally, draw solution with increased solvent concentration generated by the reverse osmosis device can be supplied from the reverse osmosis device via the container to the forward osmosis device.

According to an embodiment, the system further comprises one or more pumps fluidly coupled with the reverse osmosis device and the container, wherein the one or more pumps are configured to convey the draw solution with increased solvent concentration generated by the reverse osmosis device into the container and/or configured to convey the draw solution from the container to the reverse osmosis device. For instance, one or more drain pumps may be arranged in a drain line connecting the reverse osmosis device and the container, and configured to pump the draw solution with increased solvent concentration generated by the reverse osmosis device into the container. Alternatively or additionally, one or more supply pumps may be arranged in a supply line interconnecting the reverse osmosis device and the container, and configured to supply the draw solution from the container to the reverse osmosis device. The one or more supply pumps may also be configured to increase the pressure in the draw solution supplied to the reverse osmosis device. It should be noted, though, that also one or more pumps may be coupled between the forward osmosis device and the reverse osmosis device to transfer the draw solution between these devices directly.

According to an embodiment, the system further comprises one or more pumps fluidly coupled with the forward osmosis device and the container, wherein the one or more pumps are configured to convey the draw solution from the container to the forward osmosis device and/or to convey the draw solution with decreased solvent concentration from the forward osmosis device to the container. For instance, one or more drain pumps may be arranged in a drain line connecting the forward osmosis device and the container, and configured to pump the draw solution with decreased solvent concentration generated by the forward osmosis device into the container. Alternatively or additionally, one or more supply pumps may be arranged in a supply line interconnecting container and the forward osmosis device, and configured to supply the draw solution from the container to the forward osmosis device.

According to an embodiment, the system further comprises a wastewater container configured to receive and/or store the wastewater from the one or more dialysis devices, wherein the wastewater container is fluidly coupled with the at least one inlet. Accordingly, the wastewater container can be arranged upstream of the at least one inlet. By using a wastewater container to store the wastewater, it can be ensured that the purification process can be maintained without interruption. Also, excessive amounts of wastewater that may not be processed immediately by the system, for example when the amount of wastewater generated exceeds the amount of wastewater that can be processed by the system, can be stored temporarily in the wastewater container.

According to an embodiment, the system further comprises at least one feed pump fluidly coupled with the wastewater container and the forward osmosis device and configured to convey wastewater from the wastewater container to the forward osmosis device. Hence, an appropriate flow of wastewater to the forward osmosis device can be ensured.

According to an embodiment, the system further comprises a controller configured to control one or more of the reverse osmosis device, the forward osmosis device, one or more pumps supplying draw solution to the reverse osmosis device, one or more pumps draining draw solution from the reverse osmosis device, one or more pumps supplying draw solution to the forward osmosis device, one or more pumps draining draw solution from the forward osmosis device, and/or one or more pumps supplying wastewater to the forward osmosis device. Also one or more further components of the system can be controlled by the controller, such as one or more heating elements, one or more heating systems, one or more valves, and the like.

According to an embodiment, the system further comprises at least one outlet fluidly coupled with the reverse osmosis device and configured to supply the purified water to one or more dialysis devices. Via the at least one outlet, purified water generated by the system can be directly supplied to and/or re-used by the one or more dialysis device. Alternatively or additionally, a further purification system, for example based on reverse osmosis, can be used to further purify the water generated by the system prior to supplying it to the one or more dialysis devices. Optionally, purified water generated by the system can be stored in a container to be used at a later time by the one or more dialysis devices.

According to an embodiment, the wastewater treatment system is supplied with renewable energy. This may further allow to reduce a carbon footprint of the system and/or the purification process performed by system.

According to an embodiment, the system further comprises one or more heating elements and/or one or more heating systems with one or more heating elements arranged upstream of the forward osmosis device and configured to heat the wastewater supplied to the forward osmosis device. Alternatively or additionally, the system may further comprise one or more heating elements arranged upstream of the reverse osmosis device and configured to heat the draw solution supplied from the container to the reverse osmosis device. Alternatively or additionally, the draw solution in the container and/or the draw solution supplied to the forward osmosis device can be heated.

Generally, an efficiency or performance of the forward and the reverse osmosis devices, which can be given as amount of fluid generated by the respective device per amount supplied to it, is proportional to and/or depends on a temperature of the fluid supplied to the respective device (i.e. the wastewater and/or draw solution in case of the forward osmosis device and the draw solution in case of the reverse osmosis device). By heating the wastewater supplied to the forward osmosis device and/or by heating the draw solution supplied to the reverse osmosis device, the efficiency or performance of the forward and/or the reverse osmosis process can be increased. Further, as the purified water generated by the system may in turn also have a higher temperature, when compared to not applying a heating, an energy consumption of the dialysis device(s) can be significantly reduced, because the dialysis device(s) may not have to heat the purified water as much (or even not at all) to reach the desired body temperature of about 37°C.

In the following, various examples and exemplary embodiments of the one or more heating elements are described. The one or more heating elements may for example be part of a heating system of the wastewater treatment system. The heating system and/or the one or more heating elements may be configured to heat the wastewater supplied to the forward osmosis device and/or the draw solution supplied to the reverse/forward osmosis device(s) by at least by at least 5%, preferably at least 10%, even more preferably at least 25%, at least 50% or even more relative to the initial temperature of the wastewater and/or the draw solution (i.e. a temperature of the respective fluid when no heating is applied).

In an example, the heating system and/or the one or more heating elements may be configured to heat the wastewater supplied to the forward osmosis device and/or the draw solution supplied to the reverse/forward osmosis device(s) to a temperature of at least 15°C, preferably at least 20°C. Also higher temperatures, such as a temperature substantially corresponding to the body temperature of about 37°C or even a higher temperature, are possible.

Optionally, however, the heating system may be configured to heat the wastewater supplied to the forward osmosis device and/or the draw solution supplied to the reverse/forward osmosis device(s) to a maximum temperature of about 25°C. This can generally reduce a microbial load in the wastewater and/or the draw solution.

In an example, the heating system can include at least one heat exchanger arranged upstream of the reverse osmosis device and/or the forward osmosis device, for instance in a respective supply line. The at least one heat exchanger may be driven by and/or supplied with thermal energy via a secondary circuit and/or heating circuit, which optionally can be supplied with energy from one or more renewable energy sources. This can allow for an efficient water purification at minimum carbon footprint.

According to an example, the heating system can be configured to heat the wastewater supplied to the forward osmosis device and/or the draw solution supplied to the reverse/forward osmosis device(s) with a heating circuit, for example a secondary circuit, wherein the heating system includes at least one heating element configured to heat a heat exchange fluid of the heating circuit. In other words, a heating circuit may be provided as secondary circuit of the heating system, wherein the heat exchange fluid can be heated by the at least one heating element. The heating circuit and/or the heating system may further be configured to heat the wastewater supplied to the forward osmosis device and/or the draw solution supplied to the reverse/forward osmosis device(s), which may flow in a corresponding primary circuit based on transferring thermal energy from the heat exchange fluid to the wastewater supplied to the forward osmosis device and/or to the draw solution supplied to the reverse/forward osmosis device(s), for example based on convection and/or based on thermal coupling.

Optionally, the heating system may include at least one pump arranged in a heating circuit of the heating system, wherein the at least one pump is controllable to adjust a flow rate of a heat exchange fluid in the heating circuit. By controlling the flow rate of the heat exchange fluid, the temperature of the heat exchange fluid and in turn the temperature of the wastewater supplied to the forward osmosis device and/or the draw solution can be precisely controlled.

In yet another example, the system may further comprise a bypass line with one or more controllable valves, wherein the bypass line can be arranged upstream of the forward osmosis device and/or the reverse osmosis device and configured to bypass the heating system (or a corresponding heating element thereof) to supply non-heated water to the reverse osmosis device and/or the forward osmosis device. This can allow for a fast, efficient, reliable and precise temperature control.

The heating system, one or more heating elements thereof, a flow rate of the wastewater supplied to the forward osmosis device and/or a flow rate of the draw solution supplied to the reverse osmosis device can be controlled by a controller of the system. For example, the controller may be configured to control or regulate a temperature of a heat exchange fluid of the heating system, to operate or control at least one heating element of the heating system, and/or to operate or control one or more control valves, for example one or more valves in a bypass line and/or a heating circuit. Alternatively or additionally, the controller may be configured to control the reverse osmosis device and/or the forward osmosis device.

Optionally, the controller may receive one or more sensor signals of one or more sensors of the systems, such as temperature sensors, flow rate sensors and/or pressure sensors, to control and/or adjust the temperature of the wastewater supplied to the forward osmosis device and/or the draw solution supplied to the reverse osmosis device and/or the forward osmosis device.

In an example, the controller may be configured to control and/or adjust a temperature of the wastewater supplied to the forward osmosis device and/or the draw solution to a predetermined and/or predefined temperature. Such predefined and/or predetermined temperature may be stored in a memory or data storage of the system or may be retrieved from an external data source.

According to an embodiment, the system further comprises one or more dialysis devices fluidly coupled to the reverse osmosis device and configured to receive the purified water generated by the reverse osmosis device. The one or more dialysis devices may for example be coupled to one or more outlets of the system.

A further aspect of the present disclosure relates to a use of a system, as described hereinabove and hereinbelow, for wastewater treatment of wastewater from one or more dialysis devices.

A further aspect of the present disclosure relates to a method of treating and/or purifying wastewater from one or more dialysis devices with a system, as described hereinabove and hereinbelow. The method comprises:
- receiving, with a forward osmosis device of the system, wastewater from one or more dialysis devices;
- extracting, with the forward osmosis device, water molecules from the wastewater into a draw solution based on forward osmosis, thereby decreasing a solvent concentration in the draw solution;
- supplying the draw solution with decreased solvent concentration to a reverse osmosis device of the system; and
- extracting, with the reverse osmosis device, water molecules from the draw solution with decreased solvent concentration based on reverse osmosis, thereby increasing the solvent concentration in the draw solution and thereby generating purified water suppliable to one or more dialysis devices.

It should be noted that any feature, function, element, technical effect and/or advantage described hereinabove and hereinbelow with reference to one aspect of the present disclosure, equally applies to any other aspect of the present disclosure. In particular, any feature, function, element, technical effect and/or advantage described hereinabove and hereinbelow with reference to the wastewater system can be a feature, function, step, element, technical effect and/or advantage of the method, and vice versa.

According to an embodiment, the method further comprises supplying the draw solution with decreased solvent concentration generated by the forward osmosis device to a container of the system. Alternatively or additionally, the method comprises supplying the draw solution with increased solvent concentration generated by the reverse osmosis device to a container of the system.

According to an embodiment, the method further comprises supplying the purified water generated by the reverse osmosis device to one or more dialysis devices for dialysis treatment, for example via an outlet of the system.

These and other aspects of the invention will be apparent from and elucidated with reference to the appended figures, which may represent exemplary embodiments.

### Brief Description of the Drawings

The subject-matter of the invention will be explained in more detail in the following with reference to exemplary embodiments which are illustrated in the attached drawings, wherein:
Fig. 1 shows a wastewater treatment system according to an exemplary embodiment; and
Fig. 2 shows a flow chart illustrating steps of a method of treating wastewater according to an exemplary embodiment.

The figures are schematic only and not true to scale. In principle, identical or like parts are provided with identical or like reference symbols in the figures.

### Detailed Description of Exemplary Embodiments

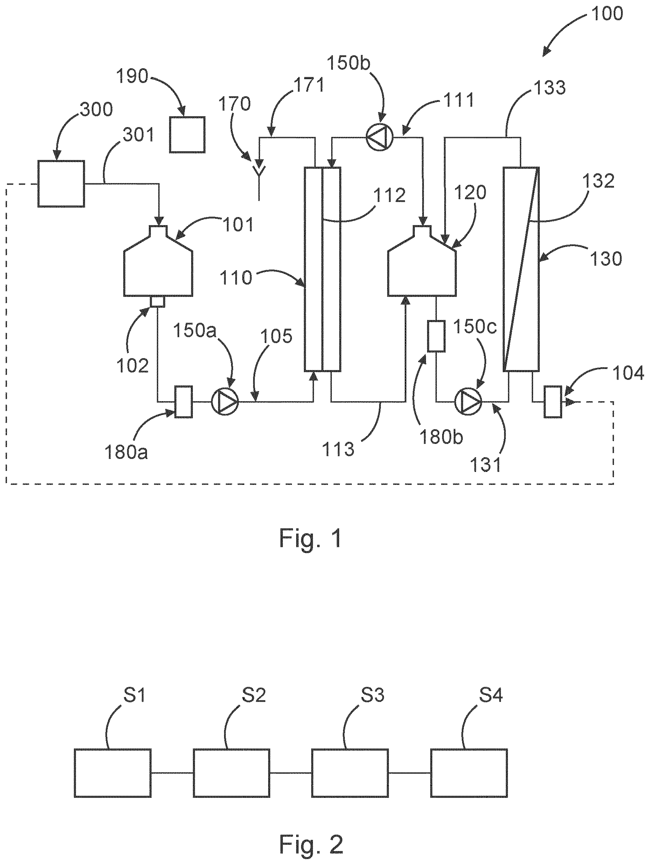

Figure 1 shows a wastewater treatment system 100 for treating and/or purifying wastewater from one or more dialysis devices 200 according to an exemplary embodiment.

The system 100 comprises at least one inlet 102 configured to receive wastewater from the one or more dialysis devices 300. In the example shown in Figure 1, the system 100 comprises an optional wastewater container 101 or tank 101 storing the wastewater from the one or more dialysis devices 300, and the inlet 102 is exemplary shown in Figure 1 to be arranged at the wastewater container 101. Alternatively, the one or more dialysis devices 300 may be directly coupled via corresponding piping or tubing to the inlet 102.

The system 100 further comprises a container 120 configured to store a draw solution containing a solvent for forward osmosis. The system 100 further comprises a forward osmosis device 110 fluidly coupled to the at least one inlet 102 to receive wastewater from the at least one inlet 102. Therein, the forward osmosis device 110 is coupled to the inlet 102 via a feed line 105, wherein one or more pumps 150a are arranged in the feed line 105 to pump wastewater from the inlet 102 or wastewater container 101 to the forward osmosis device 110.

The forward osmosis device 110 is fluidly coupled to the container 120 to receive the draw solution from the container 110. To pump and/or transport the draw solution from the container 120 to the forward osmosis device 110, the system 100 comprises one or more pumps 150b arranged in a supply line 111 interconnecting the container 120 and the forward osmosis device 110.

The system 100 further comprises a reverse osmosis device 130 fluidly coupled via a supply line 131 to the container 120. Alternatively or additionally, the forward osmosis device 110 and the reverse osmosis device 130 may be directly interconnected.

To pump and/or transport the draw solution from the container 120 to the reverse osmosis device 130, the system 100 comprises one or more pumps 150c arranged in a supply line 131 interconnecting the container 120 and the reverse osmosis device 130. By means of the one or more pumps 150c, also a pressure of the draw solution supplied to the reverse osmosis device 130 may be increased to a value sufficiently high for reverse osmosis at the reverse osmosis device 130.

The forward osmosis device 110 includes at least one semipermeable membrane 112, wherein the forward osmosis device 110 is configured to pass the wastewater received via supply line 105 along a first side of the membrane and to pass the draw solution received from the container along a second side of the membrane 112 opposite to the first side, thereby extracting water molecules from the wastewater into the draw solution through the membrane 112. Accordingly, a concentration of solvent in the draw solution may be decreased by this forward osmosis process.

Wastewater exiting the forward osmosis device 11 may be drained via a drainage 170 of the system 100 that is coupled to the forward osmosis device 170 by a corresponding drain line 171.

The draw solution with decreased solvent concentration generated by the forward osmosis device 110 is then transported into the container 120 via a corresponding drain line 113, where the draw solution in the container 120 is diluted.

Further, the reverse osmosis device 130 includes at least one semipermeable membrane 132, wherein the reverse osmosis device 130 is configured to receive the diluted draw solution from the container 120 via the supply line 131, to increase a pressure of the draw solution and pass the pressurized draw solution along a first side of the membrane 132, such that osmosis is reversed and water molecules are extracted from the pressurized draw solution through the membrane 130, thereby generating purified water. To increase the pressure of the draw solution supplied to the reverse osmosis device 130, the one or more pumps 150c may be used and/or the reverse osmosis device 130 may have another means, for example a dedicated pump, for increasing the pressure of the draw solution.

By extracting water from the draw solution based on reverse osmosis, the reverse osmosis device 130 increases the concentration of solvent in the draw solution. The draw solution with increased solvent concentration generated by the reverse osmosis device 130 can then be fed into the container 120 via a drain line 133, thereby increasing the solvent concentration of the draw solution in the container 120.

Accordingly, the decrease in solvent concentration in the draw solution caused by the forward osmosis device 110 can be compensated by the increase in solvent concentration in the draw solution generated by the reverse osmosis device 130. This can allow for a closed-loop system that maintains the forward and reverse osmosis processes.

The system 100 further comprises at least one outlet 104 fluidly coupled with the reverse osmosis device 130 and configured to supply the purified water to the one or more dialysis devices 300. Generally, this can allow for an efficient closed-loop purification process, which requires significantly less fresh water to be supplied to the system, when compared to conventional systems. In this context, it should be noted that the system 100 may further be coupled to a fresh water source for supplying fresh water to the system 100, as a minimum amount of fresh water may be required to maintain the overall purification process.

In the example shown in Figure 1, the system 100 comprises one or more heating elements 180a arranged upstream of the forward osmosis device 110 and configured to heat the wastewater to a certain temperature. This can allow to increase an efficiency of the forward osmosis device 110. Alternatively or additionally, the draw solution in the container 120 and/or the draw solution supplied from the container 120 to the forward osmosis device 110 may be heated.

The system 100 further includes one or more heating elements 180b arranged upstream of the reverse osmosis device 130 and configured to heat the draw solution supplied to the reverse osmosis device 130 to a certain temperature. This can allow to increase an efficiency of the reverse osmosis device 110.

The system 100 may further comprise a controller 190 for controlling one or more components of the system 100, such as the one or more pumps 150a, 150b, 150c, the reverse osmosis device 130, the forward osmosis device 110, one or more valves, the one or more heating elements 180a, 180b and/or other components of the system 100.

In the following, the system 100 and its functionality is briefly summarized. The wastewater from the dialysis devices 300 is fed to the wastewater container 101 via a feed line 301. The one or more pumps 150a direct the wastewater to the forward osmosis device 110 with its membrane 112. In the forward osmosis device 110, water contained in the wastewater is separated from other constituents. For this purpose, the wastewater is passed along one side of the membrane 112 and on the opposite side a higher concentrated solution, the draw solution, is passed along. This creates an osmotic pressure between the draw solution and the wastewater, which causes the water molecules to move from the wastewater into the draw solution. This draw solution is provided in the container 120 and circulated across the membrane 112 using the pump 150b.

During this process, the draw solution in the container 120 is permanently diluted by the water supplied via the forward osmosis device 110. In order to keep this process running, it may be required to concentrate the draw solution again. For this purpose, the draw solution in the container 120 is fed to the reverse osmosis device 130 with its membrane 132 by means of a pump 150c. The reverse osmosis device 130 is configured to concentrate the draw solution again and simultaneously generate the purified water. In contrast to forward osmosis, reverse osmosis uses pressure to reverse the principle of natural osmosis. Pressure is applied to the side of the membrane 132 with the higher ion (or solvent) concentrations (in this case, the draw solution), forcing the water in the opposite direction, namely to the pure water side with the lower ion or solvent concentration. As a result, the concentration on the side with the high ion or solvent concentrations increases even further and can thus be fed back into the container 120 as a draw solution. The "pure water" produced by reverse osmosis can now be further treated or fed directly to the dialysis devices 300.

Figure 2 shows a flow chart illustrating steps of a method of treating wastewater with a wastewater treatment system 100, for example the system 100 of Figure 1.

In a first step S1, wastewater is received with a forward osmosis device 110 of the system 100 from one or more dialysis devices 300.

In a further step S2, water molecules are extracted with the forward osmosis device 110 from the wastewater into a draw solution based on forward osmosis, thereby decreasing a solvent concentration in the draw solution.

Step S2 may optionally comprise supplying the draw solution with decreased solvent concentration generated by the forward osmosis device 110 to a container 120 of the system 100.

In a further step S3, the draw solution with decreased solvent concentration generated by the forward osmosis device 110 is supplied to a reverse osmosis device 130 of the system 100.

In yet another step S4, water molecules are extracted with the reverse osmosis device 130 from the draw solution with decreased solvent concentration based on reverse osmosis, thereby increasing the solvent concentration in the draw solution and thereby generating purified water suppliable to one or more dialysis devices 300.

Step S4 may optionally comprise supplying the draw solution with increased solvent concentration generated by the reverse osmosis device 130 to the container 120 of the system 100. Alternatively or additionally, step S4 may optionally comprise supplying the purified water generated by the reverse osmosis device 130 to one or more dialysis devices 300 for dialysis treatment.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practicing the claimed invention, from a study of the drawings, the disclosure, and the appended claims.

In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A wastewater treatment system (100) for treating and/or purifying wastewater from one or more dialysis devices (300), the system comprising:
at least one inlet (102) configured to receive wastewater from one or more dialysis devices;
a container (120) configured to store a draw solution containing a solvent for forward osmosis;
a forward osmosis device (110) fluidly coupled to the at least one inlet (102) to receive wastewater from the at least one inlet and fluidly coupled to the container (120) to receive the draw solution from the container; and
a reverse osmosis device (130) fluidly coupled to the container (120) and/or the forward osmosis device (110), the reverse osmosis device being configured to generate purified water based on reverse osmosis;
wherein the forward osmosis device (110) is configured to extract, based on forward osmosis using the draw solution, water molecules from the received wastewater into the draw solution, thereby decreasing a concentration of the solvent in the draw solution; and
wherein the reverse osmosis device (130) is configured to extract, based on reverse osmosis, the purified water from the draw solution with decreased solvent concentration, thereby increasing the concentration of the solvent in the draw solution.

2. The system (100) according to claim 1,
wherein the system is configured to supply the draw solution with increased solvent concentration generated by the reverse osmosis device (130) to the container (120) and/or to the forward osmosis device (110).

3. The system (100) of claim 2, further comprising:
one or more pumps (150c) fluidly coupled with the reverse osmosis device (130) and the container (120);
wherein the one or more pumps are configured to convey the draw solution with increased solvent concentration generated by the reverse osmosis device into the container and/or to convey the draw solution from the container (120) to the reverse osmosis device (130).

4. The system (100) according to any one of the preceding claims, further comprising:
one or more pumps (150b) fluidly coupled with the forward osmosis device (110) and the container;
wherein the one or more pumps (150b) are configured to convey the draw solution from the container (120) to the forward osmosis device (110) and/or to convey the draw solution with decreased solvent concentration from the forward osmosis device (110) to the container (120).

5. The system (100) according to any one of the preceding claims, further comprising:
a wastewater container (101) configured to store the wastewater from the one or more dialysis devices (300), wherein the wastewater container is fluidly coupled with the at least one inlet (102).

6. The system (100) according to claim 5, further comprising:
at least one feed pump (150a) fluidly coupled with the wastewater container (101) and the forward osmosis device (110) and configured to convey wastewater from the wastewater container to the forward osmosis device.

7. The system (100) according to any one of the preceding claims, further comprising:
at least one outlet (104) fluidly coupled with the reverse osmosis device (130) and configured to supply the purified water to one or more dialysis devices (300).

8. The system (100) according to any one of the preceding claims,
wherein the forward osmosis device (110) includes at least one semipermeable membrane (112); and
wherein the forward osmosis device is configured to pass the wastewater along a first side of the membrane and to pass the draw solution along a second side of the membrane opposite to the first side, thereby extracting water molecules from the wastewater into the draw solution through the membrane.

9. The system (100) according to any one of the preceding claims,
wherein the reverse osmosis device (130) includes at least one semipermeable membrane (132); and
wherein the reverse osmosis device is configured to receive the draw solution from the container, increase a pressure of the draw solution and pass the pressurized draw solution along a first side of the membrane, such that osmosis is reversed and water molecules are extracted from the pressurized draw solution through the membrane.

10. The system (100) according to any one of the preceding claims, further comprising:
one or more heating elements (180a,b) arranged upstream of the forward osmosis device and configured to heat the wastewater supplied to the forward osmosis device.

11. The system (100) according to any one of the preceding claims, further comprising:
one or more heating elements (180a,b) arranged upstream of the reverse osmosis device and configured to heat the draw solution supplied from the container to the reverse osmosis device.

12. The system (100) according to any one of the preceding claims, further comprising:
one or more dialysis devices (300) fluidly coupled to the reverse osmosis device and configured to receive the purified water generated by the reverse osmosis device.

13. A method of treating and/or purifying wastewater from one or more dialysis devices (300) with a system (100) according to any one of the preceding claims, the method comprising:
receiving, with a forward osmosis device (110) of the system, wastewater from one or more dialysis devices;
extracting, with the forward osmosis device, water molecules from the wastewater into a draw solution based on forward osmosis, thereby decreasing a solvent concentration in the draw solution;
supplying the draw solution with decreased solvent concentration to a reverse osmosis device (130) of the system; and
extracting, with the reverse osmosis device, water molecules from the draw solution with decreased solvent concentration based on reverse osmosis, thereby increasing the solvent concentration in the draw solution and thereby generating purified water suppliable to one or more dialysis devices.

14. The method according to claim 13, further comprising:
supplying the draw solution with decreased solvent concentration generated by the forward osmosis device (110) to a container (120) of the system; and/or
supplying the draw solution with increased solvent concentration generated by the reverse osmosis device (130) to a container (120) of the system.

15. The method according to any one of claims 13 and 14, further comprising:
supplying the purified water generated by the reverse osmosis device (130) to one or more dialysis devices (300) for dialysis treatment.
